# EUROPEAN PATENT SPECIFICATION

(11) **EP 1 945 185 B1**
(45) Date of publication and mention of the grant of the patent: **03.02.2016**
(21) Application number: 06836270.6
(22) Date of filing: 12.10.2006
(51) Int. Cl.: A61K 9/16, A61K 31/59, A61K 9/48, A61K 45/06, A61K 31/593

(54) **METHODS AND ARTICLES FOR TREATING 25-HYDROXYVITAMIN D INSUFFICIENCY AND DEFICIENCY**
VERFAHREN UND ARTIKEL ZUR BEHANDLUNG VON 25-HYDROXYVITAMIN-D-INSUFFIZIENZ UND MANGEL
PROCÉDÉS ET ARTICLES SERVANT À TRAITER L'INSUFFISANCE ET LA DÉFICIENCE EN 25-HYDROXYVITAMINE D

(30) Priority: 12.10.2005 US 725709 P
(43) Date of publication of application: 23.07.2008
(60) Divisional application: 15197974.7
(73) Proprietor: Proventiv Therapeutics, LLC, Mt. Horeb, WI 53572 (US)
(72) Inventor: BISHOP, Charles, W., Madison, WI 53572 (US); CRAWFORD, Keith, H., Fitchburg, WI 53711 (US); MESSNER, Eric, J., Lake Forest, IL 60045 (US)
(74) Representative: D Young & Co LLP
(86) International application number: PCT/US2006/039787
(87) International publication number: WO 2007/047327

(56) References cited:
- WO-A-94/00128
- DAVIES, M. ET AL: "The absorption and metabolism of vitamin D3 from parenteral injection sites" PROCEEDINGS OF THE WORKSHOP ON VITAMIN D (1979), 4TH(VITAM. D: BASIC RES. ITS CLIN. APPL.), 609-12 CODEN: PWVDDU; ISSN: 0721-7110, 1979, XP008084840
- RAPURI P B ET AL: "Effect of vitamins D2 and D3 supplement use on serum 25OHD concentration in elderly women in summer and winter." CALCIFIED TISSUE INTERNATIONAL, vol. 74, no. 2, February 2004 (2004-02), pages 150-156, XP002454820 ISSN: 0171-967X
- Alex Vasquez ET AL: "The clinical importance of vitamin D (cholecalciferol): a paradigm shift with implications for all healthcare providers", Alternative therapies in health and medicine, 1 September 2004 (2004-09-01), page 28, XP055170380, United States Retrieved from the Internet: URL:http://www.ncbi.nlm.nih.gov/pubmed/154 78784
- SHI X-Y ET AL: "Preparation of chitosan/ethylcellulose complex microcapsule and its application in controlled release of Vitamin D2", BIOMATERIALS, ELSEVIER SCIENCE PUBLISHERS BV., BARKING, GB, vol. 23, no. 23, 1 December 2002 (2002-12-01), pages 4469-4473, XP004377517, ISSN: 0142-9612, DOI: 10.1016/S0142-9612(02)00165-5
- KAJIHARA M ET AL: "NOVEL METHOD TO CONTROL RELEASE OF LIPOPHILIC DRUGS WITH HIGH POTENCY FROM SILICONE", CHEMICAL AND PHARMACEUTICAL BULLETIN, PHARMACEUTICAL SOCIETY OF JAPAN, JP, vol. 51, no. 1, 1 January 2003 (2003-01-01), pages 11-14, XP001143134, ISSN: 0009-2363, DOI: 10.1248/CPB.51.11

## Description

### BACKGROUND

### Field of the Disclosure

The disclosure relates generally to dosage forms for treating 25-hydroxyvitamin D insufficiency and/or deficiency.

### Brief Description of Related Technology

Cholecalciferol and ergocalciferol, which collectively are referred to as Vitamin D, are fat-soluble seco-steroid precursors to Vitamin D hormones that, among other activities, contribute to the maintenance of normal levels of calcium and phosphorus in the bloodstream.

Cholecalciferol and ergocalciferol are normally present at stable, low concentrations in human blood. Slight, if any increases in blood Vitamin D levels occur after meals since unsupplemented diets have low Vitamin D content, even those containing foods fortified with Vitamin D. Almost all human Vitamin D supply comes from fortified foods, exposure to sunlight or from dietary supplements, with the latter source becoming increasingly important. Blood Vitamin D levels rise only gradually, if at all, after sunlight exposure since cutaneous 7-dehydroxcholesterol is modified by UV radiation to pre- Vitamin D3, which undergoes thermal conversion in the skin to cholecalciferol over a period of several days before circulating in the blood. In contrast, supplements such as those currently available, do cause marked increases in intralumenal, blood and intracellular levels of Vitamin D proportional to the dose administered.

Both cholecalciferol and ergocalciferol are metabolized into prohormones by enzymes primarily located in the liver of the human body. Cholecalciferol is metabolized into a prohormone 25-hydroxyvitamin D₃, and ergocalciferol is metabolized into two prohormones, 25-hydroxyvitamin D₂ and 24(S)-hydroxyvitamin D₂. The two 25-hydroxylated prohormones are collectively referred to as "25-hydroxyvitamin D" ("25(OH)D"). Cholecalciferol and ergocalciferol also can be metabolized into prohormones outside of the liver in certain cells, such as enterocytes, by enzymes which are identical or similar to those found in the liver.

Elevating concentrations of either precursor increases prohormone production; similarly, lowering precursor concentrations decreases hormone production. Surges in the blood levels of cholecalciferol and/or ergocalciferol ("cholecalciferol/ergocalciferol") can transiently raise intracellular Vitamin D concentrations, accelerating prohormone production and elevating intracellular and blood prohormone concentrations. Surges in the blood levels of cholecalciferol and/or ergocalciferol also can saturate the enzymes which produce the prohormones, causing the excess Vitamin D to be catabolized or shunted to long-term storage in adipose tissue. Vitamin D stored in adipose tissue is less available for future conversion to prohormones. Surges in intralumenal levels of Vitamin D after ingestion of current oral supplements can directly boost Vitamin D and prohormone concentrations in the local enterocytes, thereby exerting "first pass" effects on calcium and phosphorus metabolism in the small intestine.

The Vitamin D prohormones are further metabolized in the kidneys into potent hormones. The prohormone 25-hydroxyvitamin D₃ is metabolized into a hormone 1α,25-dihydroxyvitamin D₃ (or calcitriol); likewise, 25-hydroxyvitamin D₂ and 24(S)-hydroxyvitamin D₂ are metabolized into hormones known as 1α,25-dihydroxyvitamin D₂ and 1α,24(S)-dihydroxyvitamin D₂ respectively. Surges in blood or intracellular prohormone concentrations can promote excessive extrarenal hormone production, leading to local adverse effects on calcium and phosphorus metabolism. Such surges also can inhibit hepatic prohormone production from subsequent supplemental Vitamin D and promote catabolism of both Vitamin D and 25-hydroxyvitamin D in the kidney and other tissues.

Blood Vitamin D hormone concentrations remain generally constant through the day in healthy individuals, but can vary significantly over longer periods of time in response to seasonal changes in sunlight exposure or sustained changes in Vitamin D intake. Normally, blood levels of cholecalciferol, ergocalciferol and the three Vitamin D prohormones are also constant through the day, given a sustained, adequate supply of Vitamin D from sunlight exposure and an unsupplemented diet. Blood levels of cholecalciferol and ergocalciferol, however, can increase markedly after administration of currently available Vitamin D supplements, especially at doses which greatly exceed the amounts needed to prevent Vitamin D deficiency rickets or osteomalacia.

The Vitamin D hormones have essential roles in human health which are mediated by intracellular Vitamin D receptors (VDR). In particular, the Vitamin D hormones regulate blood calcium levels by controlling the absorption of dietary calcium by the small intestine and the reabsorption of calcium by the kidneys. Excessive hormone levels can lead to abnormally elevated urine calcium (hypercalciuria), blood calcium (hypercalcemia) and blood phosphorus (hyperphosphatemia). The Vitamin D hormones also participate in the regulation of cellular differentiation and growth, PTH secretion by the parathyroid glands, and normal bone formation and metabolism. Further, Vitamin D hormones are required for the normal functioning of the musculoskeletal, immune and renin-angiotensin systems. Numerous other roles for Vitamin D hormones are being postulated and elucidated based on the documented presence of intracellular VDR in nearly every human tissue.

The actions of Vitamin D hormones on specific tissues depend on the degree to which they bind to (or occupy) the intracellular VDR in those tissues. Cholecalciferol and ergocalciferol have affinities for the VDR which are estimated to be at least 100-fold lower than those of the Vitamin D hormones. As a consequence, physiological concentrations of cholecalciferol and ergocalciferol exert little, if any, biological actions without prior metabolism to Vitamin D hormones. However, supraphysiologic levels of cholecalciferol and ergocalciferol, in the range of 10 to 1,000 fold higher than normal, can sufficiently occupy the VDR and exert actions like the Vitamin D hormones.

Production of Vitamin D prohormones declines when Vitamin D is in short supply, as in conditions such as Vitamin D insufficiency or Vitamin D deficiency (alternatively, hypovitaminosis D). Low production of Vitamin D prohormones leads to low blood levels of 25-hydroxyvitamin D. Inadequate Vitamin D supply often develops in individuals who are infrequently exposed to sunlight without protective sunscreens, have chronically inadequate intakes of Vitamin D, or suffer from conditions that reduce the intestinal absorption of fat soluble vitamins (such as Vitamin D). It has recently been reported that most individuals living in northern latitudes have inadequate Vitamin D supplies. Left untreated, inadequate Vitamin D supply can cause serious bone disorders, including rickets and osteomalacia.

The Institute of Medicine (IOM) of the National Academy of Sciences has concluded that an Adequate Intake (AI) of Vitamin D for a healthy individual ranges from 200 to 600 IU per day, depending on the individual's age and sex. See Standing Committee on the Scientific Evaluation of Dietary Reference Intakes, Dietary reference intakes: calcium, phosphorus, magnesium, vitamin D, and fluoride, Washington, DC: National Academy Press (1997), incorporated herein by reference. The AI for Vitamin D was defined primarily on the basis of serum 25-hydroxyvitamin D level sufficient to prevent Vitamin D deficiency rickets or osteomalacia (or at least 11 ng/mL). The IOM also established a Tolerable Upper Intake Level (UL) for Vitamin D of 2,000 IU per day, based on evidence that higher doses are associated with an increased risk of hypercalciuria, hypercalcemia and related sequelae, including cardiac arrhythmias, seizures, and generalized vascular and other soft-tissue calcification.

Currently available oral Vitamin D supplements are far from ideal for achieving and maintaining optimal blood 25-hydroxyvitamin D levels. These preparations typically contain 400 IU to 5,000 IU of Vitamin D₃ or 50,000 IU of Vitamin D₂ and are formulated for quick or immediate release in the gastrointestinal tract. When administered at chronically high doses, as is often required for Vitamin D repletion, these products have significant and, often, severe limitations which are summarized below.

High doses of immediate release Vitamin D supplements produce marked surges in blood Vitamin D levels, thereby promoting: (a) storage of Vitamin D in adipose tissue, which is undesirable because stored Vitamin D is less available for later hepatic conversion to 25-hydroxyvitamin D; (b) hepatic catabolism of Vitamin D to metabolites, which are less useful or no longer useful for boosting blood 25-hydroxyvitamin D levels, via 24- and/or 26-hydroxylation; and, (c) excessive intracellular 24- or 25-hydroxylation of Vitamin D, which leads to increased risk of hypercalciuria, hypercalcemia and hyperphosphatemia.

High doses of immediate release Vitamin D supplements also produce surges or spikes in blood and intracellular 25-hydroxyvitamin D levels, thereby promoting: (a) excessive extrarenal production of Vitamin D hormones, and leading to local aberrations in calcium and phosphorus homeostasis and increased risk of hypercalciuria, hypercalcemia and hyperphosphatemia; (b) accelerated catabolism of both Vitamin D and 25-hydroxyvitamin D by 24-and/or 26-hydroxylation in the kidney and other tissues; (c) down-regulation of hepatic production of Vitamin D prohormones, unnecessarily impeding the efficient repletion of Vitamin D insufficiency or deficiency; and, (d) local aberrations in calcium and phosphorus homeostasis mediated by direct binding to VDR.

Furthermore, high doses of immediate release Vitamin D supplements produce supraphysiologic pharmacological concentrations of Vitamin D, e.g., in the lumen of the duodenum, promoting: (a) 25-hydroxylation in the enterocytes and local stimulation of intestinal absorption of calcium and phosphorus, leading to increased risk of hypercalciuria, hypercalcemia and hyperphosphatemia; (b) catabolism of Vitamin D by 24- and 26- hydroxylation in the local enterocytes, causing decreased systemic bioavailability; and (c) absorption primarily via chylomicrons, leading to increased hepatic catabolism.

Vitamin D supplementation above the UL is frequently needed in certain individuals; however, currently available oral Vitamin D supplements are not well suited for maintaining blood 25-hydroxyvitamin D levels at optimal levels given the problems of administering high doses of immediate release Vitamin D compounds.

The international application WO94/00128 relates to compositions for increasing activated vitamin D blood level, i.e. 1,25-hydroxyvitamin D. This document discloses oral compositions for the delayed- and sustained-release of 1-alpha-hydroxy-previtamin D derivatives, having an enteric coating which is resistant to disintegration in the acidic pH of gastric juice.

### SUMMARY

One aspect of the present invention provides a composition comprising a delayed-, sustained-release oral pharmaceutical formulation comprising cholecalciferol and ergocalciferol dispersed within a solid, semi-solid, or liquid matrix, said matrix encased with an enteric coating, wherein the formulation delays substantial release of the cholecalciferol and ergocalciferol until the dosage form reaches the ileum of a patient.

In a further aspect, there is provided a composition as defined herein for use in the treatment of 25-hydroxyvitamin D insufficiency or deficiency in a patient, wherein the composition is formulated to be orally administered to a patient having 25-hydroxyvitamin D insufficiency or deficiency, as characterized by serum 25-hydroxyvitamin D levels below 30 ng/mL. The inclusion of a combination of cholecalciferol and ergocalciferol is expected to provide even further clinical benefits.

The controlled release formulation of cholecalciferol and ergocalciferol can have one or more benefits, such as significantly: increasing the bioavailability of the contained cholecalciferol/ergocalciferol by promoting absorption directly into the bloodstream rather than into the lymphatic system via chylomicrons and by reducing catabolism in the enterocytes of the upper small intestine; decreasing the undesirable first pass effects of the contained cholecalciferol/ergocalciferol on the duodenum; avoiding production of adverse supraphysiologic surges in blood levels of cholecalciferol, ergocalciferol and 25-hydroxyvitamin D; increasing the effectiveness of orally administered cholecalciferol/ergocalciferol in restoring blood concentrations of 25- hydroxyvitamin D to optimal levels (defined for patients as equal to or greater than 30 ng/mL); increasing the effectiveness of orally administered cholecalciferol/ergocalciferol in maintaining blood concentrations of 25-hydroxyvitamin D at such optimal levels; decreasing disruptions in Vitamin D metabolism and related aberrations in PTH, calcium and phosphorus homeostasis; and, decreasing the risk of serious side effects associated with Vitamin D supplementation, including hypercalciuria, hypercalcemia, hyperphosphatemia, and Vitamin D toxicity. A particular patient group contemplated is one with chronic kidney disease. Patients at stage 3, 4 and/or 5 chronic kidney disease may be treated according to the present invention.

In another aspect, the present invention provides a method of making a controlled-release formulation of cholecalciferol and/or ergocalciferol, comprising dissolving a desired quantity of cholecalciferol and/or ergocalciferol in a minimal volume of USP-grade absolute ethanol, and mixing the solution with one or more pharmaceutical-grade excipients to form a solid, semi-solid or liquid matrix which is substantially resistant to digestion in the stomach, and gradually disintegrating in the lower intestine, wherein said matrix is then encased in an enteric coating.

In another aspect, the present invention relates to a kit comprising:
(a) a stable controlled release composition comprising cholecalciferol dispersed within a solid, semi-solid, or liquid matrix, said matrix encased with an enteric coating, wherein the composition delays substantial release of the cholecalciferol until it reaches the ileum of a patient; and
(b) a stable controlled release composition comprising ergocalciferol dispersed within a solid, semi-solid, or liquid matrix, said matrix encased with an enteric coating, wherein the composition delays substantial release of the ergocalciferol until it reaches the ileum of a patient.

The foregoing brief description has outlined, in general, the featured aspects of the invention and is to serve as an aid to better understanding the more complete detailed description which is to follow.

### DETAILED DESCRIPTION

Compositions for orally dosing a subject, an animal or a human patient, in need of Vitamin D supplementation with sufficient cholecalciferol and ergocalciferol to effectively and safely restore blood 25-hydroxyvitamin D levels to optimal levels (defined for human subjects and patients as equal to or greater than 30 ng/mL) and to maintain blood 25-hydroxyvitamin D levels at such optimal levels, are described herein.

As used herein, the following definitions may be useful in aiding the skilled practitioner in understanding the invention:

As used herein, the term "substantially constant" with respect to the serum or blood level of Vitamin D means that the release profile of the controlled release (defined hereinbelow) formulation should not include increases in total serum or blood levels of cholecalciferol and ergocalciferol of greater than approximately 10 nmol/L after administration of a unit dose, optionally over a period of at least 4 hours, 12 hours, 1 day, 2 days, 3 days, 4 days, or 5 days.

As used herein, the term "substantially constant" with respect to the serum or blood level of 25-hydroxyvitamin D means that the release profile of the controlled release formulation should not include increases in total serum or blood levels of 25-hydroxyvitamin D₃ and 25-hydroxyvitamin D₂ of greater than approximately 3 ng/mL each after administration of a unit dose, optionally over a period of at least 4 hours, 12 hours, 1 day, 2 days, 3 days, 4 days, 5 days, 6 days, 1 week, 10 days, or 2 weeks.

As used herein, the term "controlled release" and "sustained release" are used interchangeably, and refer to the release of the administered Vitamin D at such a rate that total serum or blood levels of cholecalciferol, ergocalciferol and 25-hydroxyvitamin D are maintained or elevated above predosing levels for an extended period of time, e.g. 4 to 24 hours or even longer. The term "controlled release" optionally includes delayed release characteristics.

As used herein, the term "Vitamin D toxicity" is meant to refer to the side effects suffered from excessive Vitamin D intake and excessively elevated Vitamin D blood levels, including one or more of nausea, vomiting, polyuria, hypercalciuria, hypercalcemia and hyperphosphatemia.

"Supraphysiologic" in reference to intralumenal, intracellular and blood levels of Vitamin D refers to a total concentration of cholecalciferol and ergocalciferol markedly greater than the generally stable levels observed in a Vitamin D-replete subject, animal or human patient over the course of any 24-hour period by laboratory measurement when Vitamin D supplementation has been withheld for at least 30 days. "Adverse supraphysiologic surge" refers to a local or serum concentration of cholecalciferol and/or ergocalciferol, or 25-hydroxyvitamin D that elicits adverse effects such as excessive extrarenal hormone production, leading to local adverse effects on calcium and phosphorus metabolism, inhibition of hepatic 25-hydroxylation of vitamin D, increased catabolism of both Vitamin D and 25-hydroxyvitamin D, hypercalciuria, hypercalcemia and/or hyperphosphatemia, with possible cardiovascular sequelae.

"Vitamin D insufficiency and deficiency" is generally defined as having serum 25-hydroxyvitamin D levels below 30 ng/mL (see National Kidney Foundation guidelines, NKF, Am. J. Kidney Dis. 42:S1-S2O2 (2003)).

It also is specifically understood that any numerical value recited herein includes all values from the lower value to the upper value, i.e., all possible combinations of numerical values between the lowest value and the highest value enumerated are to be considered to be expressly stated in this application. For example, if a concentration range or a beneficial effect range is stated as 1% to 50%, it is intended that values such as 2% to 40%, 10% to 30%, or 1% to 3%, etc., are expressly enumerated in this specification. These are only examples of what is specifically intended.

One aspect of the disclosure includes a composition comprising a controlled release formulation of cholecalciferol and ergocalciferol and the use of such a formulation (in one embodiment, in high doses) to treat 25- hydroxyvitamin D insufficiency and deficiency at a level of efficiency heretofore unobtainable; without the undesirable first pass effects of the Vitamin D compounds on the duodenum; without adverse supraphysiological surges in intralumenal, intracellular and blood levels of cholecalciferol, ergocalciferol and 25-hydroxyvitamin D and their consequences; and without serious side effects associated with Vitamin D supplementation, namely Vitamin D toxicity.

The controlled release compositions are designed to contain concentrations of the cholecalciferol/ergocalciferol at or above the UL, and are prepared in such a manner as to effect controlled, preferably substantially constant, release of the cholecalciferol/ergocalciferol over an extended period of time. Furthermore, the compositions preferably are designed for delayed release into the ileum of the gastrointestinal tract of humans or animals. It is contemplated that in one type of embodiment the compositions will ensure a substantially constant concentration of cholecalciferol/ergocalciferol in the body and a more sustained blood level. By providing a slow and steady release of the cholecalciferol/ergocalciferol over time, blood, intralumenal and intracellular Vitamin D concentration spikes, i.e., adverse supraphysiologic levels, are mitigated or eliminated.

Compositions comprising vitamin D3 at a dose of greater than 5,000 IU, or greater than 7,500 IU, or greater than 10,000 IU are contemplated. Compositions comprising a combination of cholecalciferol and ergocalciferol at a unit dose of at least 1,500 IU (combined), or at least 2,000, 2,500, 3,000, 4,000, 5,000, 6,000, 7,000, 7,500, 8,000, 9,000, 10,000, 11,000, 12,000 or 12,500 IU are contemplated. Such unit doses less than 200,000 IU, or less than 100,000 or 75,000 or 50,000 IU are also contemplated.

The invention also contemplates that doses may be given at intervals of once a day, once every other day, three times a week, twice a week, weekly, or every 2 weeks. The cumulative dose taken each time may be 1,500 IU (cholecalciferol and ergocalciferol separately or combined), or at least 2,000, 2,500, 3,000, 4,000, 5,000, 6,000, 7,000, 7,500, 8,000, 9,000, 10,000, 11,000, 12,000 or 12,500 IU. Such doses less than 200,000 IU, or less than 100,000 or 75,000 or 50,000 IU are also contemplated. Such doses are preferred for use with adult humans.

The cholecalciferol and ergocalciferol can be included in any ratio, e.g., 9: 1 to 1 :9. Ratios including, but not limited to 1 : 1, greater than 1 : 1 cholecalciferol:ergocalciferol, and less than I :1 cholecalciferohergocalciferol, are contemplated to be useful in various embodiments.

The foregoing dosages are contemplated for oral delivery forms. The Vitamin D preparation to be administered pursuant to the use described herein can be formulated following techniques known in the art and suitable for administration via other selected routes. For example, any pharmaceutically acceptable formulation containing the preparation may be used, including, but not limited to tablets, solutions, powders, suspension, creams, aerosols, etc. Any pharmaceutically acceptable carriers known or anticipated in the art may be added to the formulation.

For example, a combination of 1,500 IU cholecalciferol and 1,500 IU ergocalciferol in a single unit dose capsule and/or in a daily dose is contemplated. Also contemplated are combinations of 1,000 IU cholecalciferol with 1,000 IU ergocalciferol in a single unit dose capsule and/or in a daily dose and 2,000 IU cholecalciferol with 2,000 IU ergocalciferol in a single unit dose capsule and/or in a daily dose. The initial dosing regimen of such a unit dose capsule can be based on baseline serum 25(OH)D (ng/ml) [nmol/L] levels, for example as detailed in Table 1 below for a combination of 1,500 IU cholecalciferol and 1,500 IU ergocalciferol in a single unit dose capsule.

**Table 1**

| Serum 25(OH)D (ng/ml) [nmol/L] | Description | Dose | Duration | Comment |
|---|---|---|---|---|
| <5 [12] | severe vitamin D deficiency | 2 capsules daily | 8 weeks | measure 25(OH)D levels |
| 5-15 [12-37] | mild vitamin D deficiency | 2 capsules daily | 6 weeks | measure 25(OH)D levels |
| 16-30 [40-75] | vitamin D insufficiency | 2 capsules daily | 2 weeks | measure 25(HO)D levels |
| ≥30 [≥75] | vitamin D sufficiency | 1 capsule daily | continuous | measure 25(OH)D levels /6 months |

To maintain serum concentrations of 25(OH)D at 30 ng/mL or above, one such capsule can be administered per day to adult patients.

The composition comprises a highly stable, controlled release pharmaceutical composition into which cholecalciferol and ergocalciferol is incorporated for convenient daily oral administration. This composition also preferably effectively resists disintegration in gastric juice, and avoids substantial release of the contained cholecalciferol and/or ergocalciferol until it reaches the small intestine, and more preferably the ileum of the small intestine. The disclosed composition produces a gradual increase in, and then sustained blood levels of, both (a) cholecalciferol and/or ergocalciferol and (b) 25-hydroxyvitamin D with dual unexpected benefits of unsurpassed effectiveness in restoring blood 25-hydroxyvitamin D to optimal levels, and unsurpassed safety relative to heretofore known oral formulations of Vitamin D. In embodiments, the use is contemplated to include administering a formulation described herein to maintain blood 25-hydroxyvitamin D levels at 30 ng/mL or higher for an extended period, for example at least one month, at least three months, at least six months, or longer.

To prepare a controlled, substantially constant release form according to the invention, cholecalciferol/ergocalciferol are dispersed within a solid, semisolid or liquid matrix, i.e. a unique mixture of rate-controlling constituents and excipients in carefully selected ratios within the matrix, and encased with a coating material. Various coating techniques can be utilized to control the rate and/or the site of the release of the cholecalciferol/ergocalciferol from the pharmaceutical formulation. For example, the dissolution of the coating may be triggered by the pH of the surrounding media, and the resulting gradual dissolution of the coating over time exposes the matrix to the fluid of the intestinal environment. After the coating becomes permeable, cholecalciferol/ergocalciferol diffuses from the outer surface of the matrix. When this surface becomes exhausted or depleted of cholecalciferol/ergocalciferol, the underlying stores begin to be depleted by diffusion through the disintegrating matrix to the external solution.

In one aspect, a formulation in accordance with the present invention provides cholecalciferol and ergocalciferol within a matrix that releasably or reversibly binds the ingredients, resulting in a controlled, substantially constant release thereof when exposed to the contents of the ileum.

The cholecalciferol- and ergocalciferol-containing matrix is suitably covered with a coating that is resistant to disintegration in gastric juices. The coated controlled release formulation of cholecalciferol/ergocalciferol is then administered orally to subjects, e.g., animals or human subjects and patients. As the formulation travels through the proximal portion of the small intestine, the enteric coating becomes progressively more permeable but, in a suitable embodiment, it provides a persisting structural framework around the cholecalciferol- and/or ergocalciferol-containing matrix. The cholecalciferol- and ergocalciferol-containing matrix becomes significantly exposed to intestinal fluids in the ileum through the permeable overcoating, and the cholecalciferol/ergocalciferol is then gradually released by simple diffusion and/or slow disintegration of the matrix.

Once released into the lumen of the ileum, the cholecalciferol/ergocalciferol is absorbed into the bloodstream. The major portion of cholecalciferol, ergocalciferol, or combination thereof when used, is absorbed at a point beyond the duodenum and jejunum. These proximal portions of the small intestine can respond to high intralumenal levels of Vitamin D and, in the process, can catabolize significant quantities of the cholecalciferol/ergocalciferol. By delaying cholecalciferol/ergocalciferol release until the ileum, the pharmaceutical composition described herein virtually eliminates first pass effects on the proximal intestine, and reduces unwanted catabolism. Further, transileal absorption of ergocalciferol can be increased with a formulation described herein, which can be designed to direct the absorbed cholecalciferol/ergocalciferol onto the serum vitamin D-binding protein (DBP) versus into chylomicrons. It is believed that cholecalciferol/ergocalciferol bound to DBP is more protected from hepatic catabolism. Significant catabolism of administered Vitamin D prior to or after its absorption into the bloodstream significantly lowers its systemic bioavailability. Elimination of first pass effects reduces the risk of Vitamin D toxicity.

In one embodiment of the invention, the controlled release formulation of cholecalciferol and ergocalciferol is prepared generally according to the following procedure. A sufficient quantity of cholecalciferol and ergocalciferol is completely dissolved in a minimal volume of USP-grade absolute ethanol (or other suitable solvent) and mixed with appropriate amounts and types of pharmaceutical-grade excipients to form a matrix which is solid or semi-solid at both room temperature and at the normal temperature of the human body. The matrix is completely or almost entirely resistant to digestion in the stomach and upper small intestine, and it gradually disintegrates in the lower intestine. In another type of embodiment a liquid matrix can be used.

In a suitable formulation, the matrix binds the cholecalciferol and ergocalciferol and permits a slow, relatively steady, preferably substantially constant, release of the cholecalciferol/ergocalciferol over a period of four to eight hours or more, by simple diffusion and/or gradual disintegration, into the contents of the lumen of the lower small intestine. The formulation further can have an enteric coating that partially dissolves in aqueous solutions having a pH of about 7.0 to 8.0, or simply dissolves slowly enough that significant release of cholecalciferol/ergocalciferol is delayed until after the formulation passes through the duodenum and jejunum.

As discussed above, the means for providing the controlled release of cholecalciferol and ergocalciferol maybe selected from any of the known controlled release delivery systems of an active ingredient over a course of about four or more hours including the wax matrix system, and the Eudragit RS/RL system (of Rohm Pharma, GmbH, Weiterstadt, Germany).

The wax matrix system provides a lipophillic matrix. The wax matrix system may utilize bees wax, white wax, cachalot wax or similar compositions. The active ingredient(s) are dispersed in the wax binder which slowly disintegrates in intestinal fluids to gradually release the active ingredient(s). The wax binder that is impregnated with the cholecalciferol and ergocalciferol is loaded into partially crosslinked soft gelatin capsules. The wax matrix system disperses the active ingredient(s) in a wax binder which softens at body temperature and slowly disintegrates in intestinal fluids to gradually release the active ingredient(s). The system suitably includes a mixture of waxes, with the optional addition of oils, to achieve a melting point which is higher than body temperature but lower than the melting temperature of gelatin formulations typically used to create the shells of either soft and/or hard gelatin capsules or other formulations used to create enteric coatings. Alternatively, the system includes a mixture of waxes, with the optional addition of oils, to remain in solid, semi-solid or liquid form at room temperature and/or body temperature.

Specifically, in one suitable embodiment, the waxes selected for the matrix are melted and thoroughly mixed. The desired quantity of oils, if any, is added at this time, followed by sufficient mixing. The waxy mixture is then gradually cooled to a temperature just above its melting point. The desired amount of cholecalciferol and ergocalciferol, dissolved in ethanol, is uniformly distributed into the molten matrix, and the matrix is loaded into soft gelatin capsules. The filled capsules are treated for appropriate periods of time with a solution containing an aldehyde, such as acetaldehyde, to partially crosslink the gelatin in the capsule shell. The gelatin shell becomes increasingly crosslinked, e.g., over a period of several weeks and, thereby, more resistant to dissolution in the contents of stomach and upper intestine. When properly constructed, this gelatin shell will gradually dissolve after oral administration and become sufficiently porous (without fully disintegrating) by the time it reaches the ileum to allow the cholecalciferol and ergocalciferol to diffuse slowly from the wax matrix into the contents of the lower small intestine.

Examples of other lipid matrices that may be of value are glycerides, fatty acids and alcohols, and fatty acid esters.

Another suitable controlled-release oral drug delivery system is the Eudragit RL/RS system in which the active ingredient, cholecalciferol and/or ergocalciferol, is formed into granules having a dimension of 25/30 mesh. The granules are then uniformly coated with a thin polymeric lacquer which is water insoluble but slowly water permeable. The coated granules can be mixed with optional additives such as antioxidants, stabilizers, binders, lubricants, processing aids and the like. The mixture may be compacted into a tablet which, prior to use, is hard and dry and can be further coated, or it may be poured into a capsule. After the tablet or capsule is swallowed and comes into contact with the aqueous intestinal fluids, the thin lacquer begins to swell and slowly allows permeation by intestinal fluids. As the intestinal fluid slowly permeates the lacquer coating, the contained cholecalciferol and ergocalciferol is slowly released. By the time the tablet or capsule has passed through the small intestine, about four to eight hours or more later, the cholecalciferol/ergocalciferol will have been slowly but completely released. Accordingly, the ingested tablet will release a stream of cholecalciferol and ergocalciferol as well as any other active ingredient.

The Eudragit system is comprised of high permeability lacquers (RL) and low permeability lacquers (RS). RS is a water insoluble film former based on neutral swellable methacrylic acids esters with a small proportion of trimetliylammonioethyl methacrylate chlorides, the molar ratio of the quaternary ammonium groups to the neural ester group being about 1 :40. RL is also a water insoluble swellable firm former based on neutral methacrylic acid esters with a small portion of trimethylammonioethyl methacrylate chloride, the molar ratio of quateranary ammonium groups to neutral ester groups being about 1:20. The permeability of the coating and thus the time course of drug release can be titrated by varying the proportion of RS to RL coating material. For further details of the Eudragit RL/RS system, reference is made to technical publications available from Rohm Tech, Inc., 195 Canal Street, Maiden, Mass. 02146. See also, K. Lehmarrn, D. Dreher "Coating of tablets and small particles with acrylic resins by fluid bed technology", Int. J. Pharm. Tech. & Prod. Mfr. 2(r), 31-43 (1981).

Other examples of insoluble polymers include polyvinyl esters, polyvinyl acetals, polyacrylic acid esters, butadiene styrene copolymers and the like.

Once the coated granules are either formed into a tablet or put into a capsule, the tablet or capsule can be coated with an enteric-coating material which dissolves at a pH of 7.0 to 8.0. One such pH dependent enteric-coating material is Eudragit L/S which dissolves in intestinal fluid but not in the gastric juices. Other enteric-coating materials may be used such as cellulose acetate phthalate (CAP) which is resistant to dissolution by gastric juices but readily disintegrates due to the hydrolytic effect of the intestinal esterases.

The particular choice of enteric-coating material and controlled release coating material can provide a controlled, preferably substantially constant release over a period of 4 to 8 hours or more, and a delayed release until the formulation reaches the ileum. Moreover, the controlled release composition in accordance with the present invention, when administered once a day, suitably provides substantially constant intralumenal, intracellular and blood Vitamin D levels, compared to an equal dose of an immediate release composition of cholecalciferol/ergocalciferol administered once a day.

The dosage forms may also contain adjuvants, such as preserving or stabilizing adjuvants. They may also contain other therapeutically valuable substances or may contain more than one of the compounds specified herein and in the claims in admixture.

Advantageously, cholecalciferol, ergocalciferol or combinations thereof together with other therapeutic agents can be orally administered in accordance with the above described embodiments. Contemplated minimum oral dosages of either vitamin, or the combination when used, include at least 200 IU per unit dose, at least 500 IU per unit dose, at least 1,500 IU per unit dose, and at least 2,000 IU per unit dose. Contemplated maximum oral dosages of either vitamin, or the combination when used, include 200,000 IU per unit dose, 50,000 IU per unit dose, 10,000 IU per unit dose, and 5,000 IU per unit dose. Contemplated oral dosage ranges of either vitamin, or the combination when used, include 200 IU per unit dose to 200,000 IU per unit dose, 500 IU per unit dose to 50,000 IU per unit dose, 1,500 IU per unit dose to 10,000 IU per unit dose, and 2,000 IU per unit dose to 5,000 IU per unit dose. Preferably the dosage form will be administered daily, such that the foregoing dosages also correspond to the equivalent values of IU per day. If the compounds of the present invention are administered in combination with other therapeutic agents, the proportions of each of the compounds in the combination being administered will be dependent on the particular disease state being addressed. For example, one may choose to administer cholecalciferol and/or ergocalciferol with one or more calcium salts (intended as a calcium supplement or dietary phosphate binder), bisphosphonates, calcimimetics, nicotinic acid, iron, phosphate binders, active Vitamin D sterols, 25-hydroxyvitamin D, inhibitors of CYP24 expression or activity, glycemic and hypertension control agents, and various antineoplastic agents. In practice, higher doses of the compounds of the present invention are used where therapeutic treatment of a disease state (e.g., chronic kidney disease) is the desired end, while the lower doses are generally used for prophylactic purposes, it being understood that the specific dosage administered in any given case will be adjusted in accordance with the specific compounds being administered, the disease to be treated, the condition of the subject and the other relevant medical facts that may modify the activity of the drug or the response of the subject, as is well known by those skilled in the art.

The inclusion of a combination of cholecalciferol and ergocalciferol in the controlled-release oral drug delivery system allows the resulting formulation to be useful in supporting both the Vitamin D₃ and Vitamin D₂ endocrine systems. Currently available oral Vitamin D supplements support just one or the other system.

### EXAMPLES

The present invention is further explained by the following examples.

### Example 1

### One embodiment of a controlled release formulation

Purified yellow beeswax and fractionated coconut oil are combined in a ratio of 1:1 and heated with continuous mixing to 75 degrees Celsius until a uniform mixture is obtained. The wax mixture is continuously homogenized while cooled to approximately 45 degrees Celsius. Cholecalciferol and ergocalciferol, in a ratio of 1:1, are dissolved in absolute ethanol and the ethanolic solution is added, with continuous homogenization, to the molten wax mixture. The amount of ethanol added is in the range of 1 to 2 v/v%. Mixing is continued until the mixture is uniform. The uniform mixture is loaded into soft gelatin capsules. The capsules are immediately rinsed to remove any processing lubricant(s) and briefly immersed in an aqueous solution of acetaldehyde in order to crosslink the gelatin shell. The concentration of the acetaldehyde solution and the immersion time is selected to achieve crosslinking to the desired degree, as determined by near-infrared spectrophotometry. The finished capsules are washed, dried and packaged.

### Example 2

### Pharmacokinetics Testing in Dogs

Twenty male beagle dogs are divided randomly into two comparable groups and receive no supplemental Vitamin D for the next 30 days. At the end of this time, each dog in Group #1 receives a single soft gelatin capsule containing 10,000 IU of ergocalciferol prepared in a controlled release formulation similar to the one disclosed in Example 1. Each dog in the other group (Group #2) receives a single immediate-release soft gelatin capsule containing 10,000 IU of ergocalciferol dissolved in medium chain triglyceride oil. All dogs receive no food for at least 8 hours prior to dosing. Blood is drawn from each dog at 0, 0.5, 1, 1.5, 2, 3, 4, 6, 9, 15, 24, 36, and 72 hours after dose administration. The collected blood is analyzed for the contained levels of ergocalciferol and 25-hydroxyvitamin D, and the data are analyzed by treatment group. Dogs in Group #1 show a slower rise and a lower maximum (Cₘₐₓ) in mean blood levels of ergocalciferol and 25-hydroxyvitamin D than dogs in Group #2. However, dogs in Group #1 show a more prolonged elevation of mean blood levels of ergocalciferol and 25-hydroxyvitamin D₂ relative to dogs in Group #2, despite that the Cₘₐₓ in Group #1 is lower. The mean area under the curve (AUC), corrected for predose background levels (recorded at t=0), is substantially greater for Group #1 for both ergocalciferol and 25-hydroxyvitamin D. These procedures will demonstrate that administration of ergocalciferol in the formulation described herein to dogs will result in blood levels of ergocalciferol and 25-hydroxyvitamin D which rise much more gradually and remain more stable than after dosing with the same amount of ergocalciferol formulated for immediate release (in medium chain triglyceride oil). The greater AUC for blood levels of ergocalciferol in Group #1 demonstrate that the bioavailability of ergocalciferol formulated as described herein is markedly improved. The greater AUC for blood levels of 25-hydroxyvitamin D in Group # 1 demonstrates that the ergocalciferol formulated as described herein is more efficiently converted to 25-hydroxyvitamin D. The difference is believed to be based on higher bioavailability and reduced catabolic clearance. It is anticipated that the elimination or reduction in post-dosing "spikes" in blood levels of ergocalciferol and 25- hydroxyvitamin D by use of a formulation described herein reduces the induction of CYP24 and, thereby, reduces unwanted catabolism of 25-hydroxyvitamin D. Thus, one of the benefits exhibited by the compositions and methods of the invention may be a reduced induction of circulating CYP24 or CYP24 in liver, kidney or intestine as measured, e.g., by mRNA or protein levels in the tissue.

Further, transileal absorption of ergocalciferol can be increased with a formulation described herein, which can be designed to direct the absorbed ergocalciferol onto the serum vitamin D-binding protein (DBP) versus into chylomicrons. It is believed that ergocalciferol bound to DBP is more protected from hepatic catabolism. Still further, it is expected that a mixture of ergocalciferol and cholecalciferol totaling 10,000 IU would be even less prone to catabolism than 10,000 IU of ergocalciferol or cholecalciferol alone. Without intending to be bound by any particular theory, this expectation is based on the different metabolic and catabolic pathways associated with ergocalciferol and cholecalciferol, and that dividing the dose between the vitamins (and preferably using a controlled-release formulation) can help protect both vitamins from CYP24 and other catabolic enzymes.

### Example 3

### Pharmacokinetics Testing in Healthy Normal Volunteers

Sixteen healthy non-obese adults, aged 18 to 24 years, participate in an 11-week pharmacokinetic study in which they receive successively, and in a double-blinded fashion, two formulations of ergocalciferol. One of the formulations (Formulation #1) is a soft gelatin capsule containing 50,000 IU of ergocalciferol prepared in a controlled release formulation similar to the one disclosed in Example 1. The other formulation (Formulation #2) is an immediate-release soft gelatin capsule of identical appearance containing 50,000 IU of ergocalciferol dissolved in medium chain triglyceride oil. For 60 days prior to study start and continuing through study termination, the subjects abstain from taking other Vitamin D supplements. On Days 1, 3 and 5 of the study, all subjects provide fasting morning blood samples to establish pre-treatment baseline values. On the morning of Day 8, the subjects provide an additional fasting blood sample (t=0), are randomly assigned to one of two treatment groups. Both groups are dosed with a single test capsule prior to eating breakfast: one group receives a capsule of Formulation #1 and the other group receives a capsule of Formulation #2. Blood is drawn from each subject at 0.5, 1, 1.5, 2, 3, 4, 6, 8, 10, 12, 15, 24, 36, 48, 72 and 108 hours after dose administration. On the morning of Day 70, the subjects provide fasting morning blood samples (t=0) and are dosed with a single capsule of the other test formulation prior to eating breakfast. Blood is again drawn from each subject at 0.5, 1, 1.5, 2, 3, 4, 6, 8, 10, 12, 15, 24, 36, 48, 72 and 108 hours after dose administration. All collected blood is analyzed for the contained levels of ergocalciferol and 25-hydroxyvitamin D, and the data are analyzed by treatment formulation after correction for baseline content. Formulation #1 is found to produce a slower rise and a lower Cₘₐₓ in mean blood levels of ergocalciferol and 25-hydroxyvitamin D than Formulation #2. However, Formulation #1 also produces a more prolonged elevation of mean blood levels of ergocalciferol and 25-hydroxyvitamin D₂ relative to Formulation #2, despite that the recorded Cₘₐₓ is lower. The mean AUC is substantially greater after administration of Formulation #1 for both ergocalciferol and 25-hydroxyvitamin D. These procedures can demonstrate that administration of ergocalciferol in the formulation described in this invention to healthy human adults results in blood levels of ergocalciferol and 25-hydroxyvitamin D which rise much more gradually and remain more stable than after dosing with the same amount of ergocalciferol formulated for immediate release (in medium chain triglyceride oil). The greater AUC for blood levels of Vitamin D after dosing with Formulation #1 demonstrate that the bioavailability of ergocalciferol formulated as described herein is better. The greater AUC for blood levels of 25-ydroxyvitamin D after Formulation # 1 demonstrate that the ergocalciferol formulated as described herein is more efficiently converted to 25-hydroxyvitamin D.

### Example 4

### Efficacy Study in Healthy Adult Male Volunteers With Vitamin D Insufficiency

The effectiveness of three different formulations of Vitamin D in restoring serum 25-hydroxyvitamin D to optimal levels (greater than 30 ng/mL) is examined in a 23-day study of healthy non-obese men diagnosed with Vitamin D insufficiency. One of the formulations (Formulation #1) is a soft gelatin capsule containing 2,500 IU of Vitamin D, comprised of a mixture of 1,250 IU of cholecalciferol and 1,250 IU of ergocalciferol and prepared as described herein. The second formulation (Formulation #2) is an immediate-release soft gelatin capsule of identical appearance containing 50,000 IU of ergocalciferol dissolved in medium chain triglyceride oil. The third formulation (Formulation #3) is an immediate-release soft gelatin capsule, also of identical appearance, containing 50,000 IU of cholecalciferol dissolved in medium chain triglyceride oil. A total of 100 healthy Caucasian and African-American men participate in this study, all of whom are aged 30 to 45 years and have serum 25-hydoxyvitamin D levels between 15 and 29 ng/mL (inclusive). All subjects abstain from taking other Vitamin D supplements for 60 days before study start and continuing through study termination. On Day 1 and 2 of the study, all subjects provide fasting morning blood samples to establish pre-treatment baseline values of serum 25-hydroxyvitamin D. On the morning of Day 3, the subjects provide an additional fasting blood sample (t=0), are randomly assigned to one of four treatment groups, and are dosed with a single test capsule prior to eating breakfast: the subjects in Group #1 each receive a single capsule of Formulation #1, and the subjects in Groups #2 and #3 each receive a single capsule of Formulation #2 and Formulation #3, respectively. Subjects in Group #4 receive a matching placebo capsule. Subjects in Group #1 each receive an additional capsule of Formulation #1 on the mornings of Days 4 through 22 before breakfast, but subjects in Groups #2, #3 and #4 receive no additional capsules. Subjects in Groups #1, #2 and #3 receive a total dose of 50,000 IU of Vitamin D over the course of the study. A fasting morning blood sample is drawn from each subject, irrespective of treatment group, on Days 4, 5, 6, 10, 17 and 23 (or 1, 2, 3, 7, 14 and 20 days after the start of dosing). All collected blood is analyzed for the contained levels of Vitamin D and 25-hydroxyvitamin D, and the data are analyzed by treatment group after correction for baseline values. Subjects in all four treatment groups exhibit mean baseline serum total Vitamin D levels of approximately 8 to 10 nmol/L and baseline serum 25-hydoxyvitamin D levels of approximately 16 to 18 ng/mL, based on analysis of fasting blood samples drawn on Days 1 through 3. Subjects in Group #4 (control group) show no significant changes in either mean serum Vitamin D or mean serum 25-hydroxyvitamin D over the course of the study. Subjects in Group #1 show mean increases in serum Vitamin D in the range of approximately 2-5 nmol/L during the course of the study, and a steadily increasing mean serum 25-hydroxyvitamin D reaching approximately 37 ng/mL by Day 23. In marked contrast, subjects in Groups #2 and #3 show mean increases in blood Vitamin D of more than 25 nmol/L by 24 hours after dosing, followed by decreases toward baseline levels over the following week, reaching baseline levels well before study end. Subjects in Group #2 exhibit increases in mean serum 25-hydroxyvitamin D for the first few days post-dosing, reaching a maximum of just above 25 ng/mL, and then rapidly declining thereafter. By study end, serum 25-hydroxyvitamin D is significantly lower than baseline in Group #2. Subjects in Group #3 exhibit continuing increases in mean serum 25-hydroxyvitamin D through the first 2 weeks after dosing with gradual, but progressive, decreases occurring thereafter. By study end, mean serum 25-hydroxyvitamin D is below 30 ng/mL, being only approximately 11 ng/mL higher than pre-treatment baseline. The data from this study can demonstrate that administration of 50,000 IU of Vitamin D, formulated as described herein and administered at a daily dose of 2,500 IU per day for 20 days, is substantially more effective in restoring low serum levels of 25-hydroxyvitamin D to optimal levels than immediate-release formulations of 50,000 IU of either ergocalciferol or cholecalciferol administered in single doses, as currently recommended by the NKF and other leading experts on oral Vitamin D replacement therapy.

### Example 5

### Efficacy and Safety Study in Healthy Postmenopausal Volunteers Exhibiting Vitamin D Deficiency

The efficacy and safety of two different formulations of Vitamin D in restoring serum 25-hydroxyvitamin D to optimal levels (greater than 30 ng/mL) are examined in a 1-year study of healthy non-obese postmenopausal women diagnosed with Vitamin D insufficiency. One of the formulations (Formulation #1) is a soft gelatin capsule containing 1,000 IU of cholecalciferol, prepared in a controlled release formulation similar to the one disclosed in Example 1. The second formulation (Formulation #2) is an immediate-release soft gelatin capsule of identical appearance containing 1,000 IU of cholecalciferol dissolved in medium chain triglyceride oil. A total of 350 healthy Caucasian, Asian, Hispanic and African-American women participate in this study, all of whom are at least 5 years postmenopausal and have serum 25-hydoxyvitamin D levels below 15 ng/mL. Prior to enrolling, all subjects provide two fasting morning blood samples and two 24-hour urine collections, separated by at least one week, to establish pre-treatment baseline values of serum calcium, plasma intact PTH, serum 25-hydroxyvitamin D, and 24-hour urine calcium excretion. On the morning of Day 1, the subjects are randomly assigned to one of seven treatment groups, and they begin daily dosing with one of the two test preparations, or with a matching placebo. Three of the treatment groups self-administer one, two or 4 capsules/day, respectively, of Formulation #1 and three other groups self-administer one, two or 4 capsules/day, respectively, of Formulation #2. The remaining treatment group self-administers one placebo capsule per day. All dosing occurs in the morning before breakfast. Additional fasting blood samples and 24-hour urine collections are obtained from each subject at quarterly intervals for determination of serum calcium, plasma intact PTH, serum 25-hydroxyvitamin D, and 24-hour urine calcium excretion. Throughout the study, all subjects adhere to a daily intake of approximately 1,000 to 1,500 mg of elemental calcium (from self-selected diets and calcium supplements, as needed) under the ongoing guidance of a dietician. At the conclusion of the study, the laboratory data are analyzed by treatment group and by test formulation after appropriate correction for baseline values. All seven groups have comparable mean baseline values for serum 25-hydroxyvitamin D (range: 10.7 ng/mL to 11.9 ng/mL), plasma intact PTH (range: 45.3 pg/mL to 52.1 pg/mL), serum calcium (range: 9.15 mg/dL to 9.31 mg/dL), and 24-hour urine calcium (range: 55 mg/day to 64 mg/day). No significant changes in any of the laboratory mean values are observed in the placebo (control) group over the course of the study. Subjects in the three treatment groups receiving Formulation #1 and in the three treatment groups receiving Formulation #2 exhibit progressively increasing serum 25-hydroxyvitamin D levels during the first 6 months of dosing, reaching steady state levels thereafter. Analysis of the mean increase in serum 25-hydroxyvitamin D vs daily dose at the end of the study shows near linear direct dose-response relationships for both formulations; however, the slope of the relationship for Formulation #1 is significantly greater than that for Formulation #2. Analysis of mean change in plasma intact PTH vs. daily dose at 6, 9 and 12 months reveals non-linear inverse relationships for both formulations, with intact PTH decreasing more at the highest dose of Formulation #2 than at the highest dose of Formulation #1. Mean serum calcium and mean 24-hour urine calcium increase significantly from baseline in all treatment groups receiving Vitamin D, and are significantly higher on the higher doses of Formulation #2 than on the comparable doses of Formulation #1. Episodes of hypercalciuria, defined as 24-hour urine calcium above 250 mg, and hypercalcemia, defined as serum calcium above 9.5 mg/dL, are observed in significantly more of subjects treated with highest dose of Formulation #2 compared with the highest dose of Formulation #1. Data from this study can demonstrate that Formulation #1 is more effective at increasing serum 25-hydroxyvitamin D than Formulation #2, and that Formulation #1 causes far fewer side effects related to calcium and PTH metabolism.

### Example 6

Sixteen healthy non-obese adults, aged 18 to 24 years, participate in a pharmacokinetic study in which they receive successively, and in a double-blinded fashion, two formulations. One of the formulations is a soft gelatin capsule containing a combination of 1,500 IU of cholecalciferol and 1,500 IU of ergocalciferol (Formulation #1) prepared in a controlled-release formulation. The other formulation is an immediate-release soft gelatin capsule of identical appearance containing a combination of 1,500 IU of cholecalciferol and 1,500 IU of ergocalciferol (Formulation #2) dissolved in medium chain triglyceride oil. For 60 days prior to study start and continuing through study termination, the subjects abstain from taking other Vitamin D supplements. On Days 1, 3 and 5 of the study, all subjects provide fasting morning blood samples to establish pre-treatment baseline values. On the morning of Day 8, the subjects provide an additional fasting blood sample (t=0), are randomly assigned to one of two treatment groups. Both groups are dosed with a single test capsule prior to eating breakfast: one group receives a capsule of Formulation #1 and the other group receives a capsule of Formulation #2. Blood is drawn from each subject at 0.5, 1, 1.5, 2, 3, 4, 6, 8, 10, 12, 15, 24, 36, 48, 72 and 108 hours after dose administration. All collected blood is analyzed for the contained levels of cholecalciferol, ergocalciferol, and 25-hydroxyvitamin D, and the data are analyzed by treatment formulation after correction for baseline content.

The efficacy and safety of the controlled-release formulation for the prevention and treatment of hypovitaminosis D in patients with chronic kidney disease (restoring serum total 25-hydroxyvitamin D to targeted levels (≥ 30 ng/mL)) are examined in a 2-month study. A total of 100 healthy Caucasian and African-American men participate in this study, all of whom are aged 30 to 45 years and have serum 25-hydoxyvitamin D levels between 15 and 29 ng/mL (inclusive). All subjects abstain from taking other Vitamin D supplements for 60 days before study start and continuing through study termination. On Day 1 and 2 of the study, all subjects provide fasting morning blood samples to establish pre-treatment baseline values of serum 25-hydroxyvitamin D. On the morning of Day 3, the subjects provide an additional fasting blood sample (t=0), are randomly assigned to one of two treatment groups, and are dosed with a single test capsule prior to eating breakfast: the subjects in Group #1 each receive a single capsule of Formulation #1, and the subjects in Group #2 each receive a single capsule of Formulation #2. Subjects in Group #1 each receive an additional capsule of Formulation #1 on the mornings of Days 4 through 22 before breakfast, but subjects in Groups #2 receive no additional capsules. A fasting morning blood sample is drawn from each subject, irrespective of treatment group, on Days 4, 5, 6, 10, 17 and 23 (or 1, 2, 3, 7, 14 and 20 days after the start of dosing). All collected blood is analyzed for the contained levels of Vitamin D and 25-hydroxyvitamin D, and the data are analyzed by treatment group after correction for baseline values.

## Claims

1. A composition comprising a delayed-, sustained-release oral pharmaceutical formulation comprising cholecalciferol and ergocalciferol dispersed within a solid, semi-solid, or liquid matrix, said matrix encased with an enteric coating, wherein the formulation delays substantial release of the cholecalciferol and ergocalciferol until the dosage form reaches the ileum of a patient.

2. The composition of claim 1, wherein the formulation releases the cholecalciferol and ergocalciferol at a substantially constant rate.

3. The composition of claim 2, wherein the formulation releases the cholecalciferol and ergocalciferol at a substantially constant rate over a period of at least four hours.

4. The composition of any one of the preceding claims, comprising at least 500 IU total of cholecalciferol and ergocalciferol.

5. The composition of any one of the preceding claims, comprising at least 1,500 IU total of cholecalciferol and ergocalciferol.

6. The composition of any one of the preceding claims, comprising at least 2,000 IU total of cholecalciferol and ergocalciferol.

7. The composition of any one of the preceding claims, comprising less than 50,000 IU total of cholecalciferol and ergocalciferol.

8. The composition of any one of the preceding claims, comprising 10,000 IU or less total of cholecalciferol and ergocalciferol.

9. The composition of any one of the preceding claims, comprising 5,000 IU or less total of cholecalciferol and ergocalciferol.

10. The composition of any one of claims 1 to 3, comprising a total of amount of cholecalciferol and ergocalciferol in a range of 500 IU to 50,000 IU.

11. The composition of any one of claims 1 to 3, comprising 1,500 IU cholecalciferol and 1,500 IU ergocalciferol.

12. The composition of any one of claims 1 to 10, wherein the enteric coating is resistant to disintegration in gastric juices and partially dissolves at a pH in a range of 7.0 to 8.0.

13. The composition of any one of claims 1-11, wherein the enteric coating comprises cellulose acetate phthalate.

14. The composition of any one of claims 1-11, further comprising a calcimimetic agent.

15. The composition of any one of claims 1-11, wherein the matrix is a wax matrix.

16. The composition of claim 15, wherein the wax matrix comprises a mixture of waxes, optionally with the addition of oils.

17. A method of making a controlled-release formulation of cholecalciferol and/or ergocalciferol, comprising dissolving a desired quantity of cholecalciferol and/or ergocalciferol in a minimal volume of USP-grade absolute ethanol, and mixing the solution with one or more pharmaceutical-grade excipients to form a solid, semi-solid or liquid matrix which is substantially resistant to digestion in the stomach, and gradually disintegrating in the lower intestine, wherein said matrix is then encased in an enteric coating.

18. The composition of any one of claims 1 to 11, for use in the treatment of 25-hydroxyvitamin D insufficiency or deficiency in a patient, wherein the composition is formulated to be orally administered to a patient having 25-hydroxyvitamin D insufficiency or deficiency, as **characterized by** serum 25-hydroxyvitamin D levels below 30 ng/mL.

19. The composition for use of claim 18, wherein the total serum levels of cholecalciferol and ergocalciferol are increased in an amount of 10 nmol/L or less over a period of at least 2 days.

20. The composition for use of claim 18 or 19, wherein the total serum levels of 25-hydroxyvitamin D₃ and 25-hydroxyvitamin D₂ are increased in an amount of 3 ng/mL or less combined over a period of at least 2 days.

21. The composition for use of any one of claims 18 to 20, wherein serum 25-hydroxyvitamin D levels are restored to at least 30 ng/mL.

22. The composition for use of any one of claims 18 to 21, wherein serum 25-hydroxyvitamin D levels are maintained at least 30 ng/mL for a period of at least three months.

23. The composition for use of claim 22, wherein serum 25-hydroxyvitamin D levels are maintained at least 30 ng/mL for a period of at least six months.

24. The composition for use of any one of claims 18 to 23, further comprising coadministering a calcimimetic agent.

25. A kit comprising:
(a) a stable controlled release composition comprising cholecalciferol dispersed within a solid, semi-solid, or liquid matrix, said matrix encased with an enteric coating, wherein the composition delays substantial release of the cholecalciferol until it reaches the ileum of a patient; and
(b) a stable controlled release composition comprising ergocalciferol dispersed within a solid, semi-solid, or liquid matrix, said matrix encased with an enteric coating, wherein the composition delays substantial release of the ergocalciferol until it reaches the ileum of a patient.

## Patentansprüche

1. Zusammensetzung, umfassend eine orale pharmazeutische Formulierung mit verzögerter/anhaltender Freisetzung, umfassend Cholecalciferol und Ergocalciferol dispergiert in einer festen, halbfesten oder flüssigen Matrix, wobei die Matrix von einem magensaftresistenten Überzug umhüllt ist, wobei die Formulierung eine wesentliche Freisetzung des Cholecalciferols und des Ergocalciferols verzögert, bis die Dosierungsform das Ileum eines Patienten erreicht.

2. Zusammensetzung nach Anspruch 1, wobei die Formulierung das Cholecalciferol und das Ergocalciferol mit einer im Wesentlichen konstanten Rate freisetzt.

3. Zusammensetzung nach Anspruch 2, wobei die Zusammensetzung das Cholecalciferol und das Ergocalciferol mit einer im Wesentlichen konstanten Rate über einen Zeitraum von mindestens 4 Stunden freisetzt.

4. Zusammensetzung nach einem der vorhergehenden Ansprüche, umfassend mindestens 500 IU Cholecalciferol und Ergocalciferol insgesamt.

5. Zusammensetzung nach einem der vorhergehenden Ansprüche, umfassend mindestens 1 500 IU Cholecalciferol und Ergocalciferol insgesamt.

6. Zusammensetzung nach einem der vorhergehenden Ansprüche, umfassend mindestens 2 000 IU Cholecalciferol und Ergocalciferol insgesamt.

7. Zusammensetzung nach einem der vorhergehenden Ansprüche, umfassend weniger als 50 000 IU Cholecalciferol und Ergocalciferol insgesamt.

8. Zusammensetzung nach einem der vorhergehenden Ansprüche, umfassend 10 000 IU oder weniger Cholecalciferol und Ergocalciferol insgesamt.

9. Zusammensetzung nach einem der vorhergehenden Ansprüche, umfassend 5 000 IU oder weniger Cholecalciferol und Ergocalciferol insgesamt.

10. Zusammensetzung nach einem der Ansprüche 1 bis 3, umfassend eine Gesamtmenge an Cholecalciferol und Ergocalciferol im Bereich von 500 IU bis 50 000 IU.

11. Zusammensetzung nach einem der Ansprüche 1 bis 3, umfassend 1 500 IU Cholecalciferol und 1 500 IU Ergocalciferol.

12. Zusammensetzung nach einem der Ansprüche 1 bis 10, wobei der magensaftresistente Überzug gegenüber einem Zerfall in Magensäften resistent ist und sich bei einem pH-Wert im Bereich von 7,0 bis 8,0 teilweise auflöst.

13. Zusammensetzung nach einem der Ansprüche 1-11, wobei der magensaftresistente Überzug Celluloseacetatphthalalat umfasst.

14. Zusammensetzung nach einem der Ansprüche 1-11, welche weiterhin ein calcimimetisches Mittel umfasst.

15. Zusammensetzung nach einem der Ansprüche 1-11, wobei es sich bei der Matrix um eine Wachsmatrix handelt.

16. Zusammensetzung nach Anspruch 15, wobei die Wachsmatrix eine Wachsmischung, gegebenenfalls unter Zusatz von Ölen, umfasst.

17. Verfahren zur Herstellung einer Formulierung von Cholecalciferol und/oder Ergocalciferol mit kontrollierter Freisetzung, bei dem man eine gewünschte Menge an Cholecalciferol und/oder Ergocalciferol in einem Mindestvolumen von absolutem Ethanol mit USP-Qualität löst und die Lösung mit einem oder mehreren Exzipienten pharmazeutischer Qualität mischt, unter Bildung einer festen, halbfesten oder flüssigen Matrix, die gegenüber einem Verdau im Magen im Wesentlichen resistent ist und im unteren Darm schrittweise zerfällt, wobei die Matrix dann mit einem magensaftresistenten Überzug umhüllt wird.

18. Zusammensetzung nach einem der Ansprüche 1 bis 11 zur Verwendung bei der Behandlung von 25-Hydroxyvitamin-D-Insuffizienz oder -Mangel bei einem Patienten, wobei die Zusammensetzung für die orale Verabreichung an einen Patienten mit 25-Hydroxyvitamin-D-Insuffizienz bzw. -Mangel, charakterisiert durch 25-Hydroxyvitamin-D-Serumkonzentrationen unter 30 ng/ml, formuliert ist.

19. Zusammensetzung zur Verwendung nach Anspruch 18, wobei die Serumgesamtkonzentrationen an Cholecalciferol und Ergocalciferol über einen Zeitraum von mindestens zwei Tagen um eine Menge von 10 nmol/l oder weniger erhöht werden.

20. Zusammensetzung zur Verwendung nach Anspruch 18 oder 19, wobei die Serumgesamtkonzentrationen an 25-Hydroxyvitamin D₃ und 25-Hydroxyvitamin D₂ kombiniert über einen Zeitraum von mindestens 2 Tagen um eine Menge von 3 ng/ml oder weniger erhöht werden.

21. Zusammensetzung zur Verwendung nach einem der Ansprüche 18 bis 20, wobei die 25-Hydroxyvitamin-D-Serumkonzentrationen auf mindestens 30 ng/ml zurückgeführt werden.

22. Zusammensetzung zur Verwendung nach einem der Ansprüche 18 bis 21, wobei die 25-Hydroxyvitamin-D-Serumkonzentrationen über einen Zeitraum von mindestens 3 Monaten auf mindestens 30 ng/ml gehalten werden.

23. Zusammensetzung zur Verwendung nach Anspruch 22, wobei die 25-Hydroxyvitamin-D-Serumkonzentrationen über einen Zeitraum von mindestens 6 Monaten auf mindestens 30 ng/ml gehalten werden.

24. Zusammensetzung zur Verwendung nach einem der Ansprüche 18 bis 23, weiterhin umfassend die gleichzeitige Verabreichung eines calcimimetischen Mittels.

25. Kit, umfassend:
(a) eine stabile Zusammensetzung mit kontrollierter Freisetzung, welche Cholecalciferol dispergiert in einer festen, halbfesten oder flüssigen Matrix umfasst, wobei die Matrix von einem magensaftresistenten Überzug umhüllt ist, wobei die Zusammensetzung eine wesentliche Freisetzung des Cholecalciferols verzögert, bis sie das Ileum eines Patienten erreicht; und
(b) eine stabile Zusammensetzung mit kontrollierter Freisetzung, welche Ergocalciferol dispergiert in einer festen, halbfesten oder flüssigen Matrix umfasst, wobei die Matrix von einem magensaftresistenten Überzug umhüllt ist, wobei die Zusammensetzung eine wesentliche Freisetzung des Ergocalciferols verzögert, bis sie das Ileum eines Patienten erreicht.

## Revendications

1. Composition comprenant une formulation pharmaceutique orale à libération retardée prolongée comprenant du cholécalciférol et de l'ergocalciférol dispersés dans une matrice solide, semi-solide ou liquide, ladite matrice étant confinée avec un enrobage entérique, la formulation retardant la libération substantielle du cholécalciférol et de l'ergocalciférol jusqu'à ce que la forme pharmaceutique atteigne l'iléon d'un patient.

2. Composition de la revendication 1, la formulation libérant le cholécalciférol et ergocalciférol à un taux sensiblement constant.

3. Composition de la revendication 2, la formulation libérant le cholécalciférol et l'ergocalciférol à un taux sensiblement constant sur une période d'au moins quatre heures.

4. Composition de l'une quelconque des revendications précédentes, comprenant au moins 500 UI au total de cholécalciférol et d'ergocalciférol.

5. Composition de l'une quelconque des revendications précédentes, comprenant au moins 1 500 UI au total de cholécalciférol et d'ergocalciférol.

6. Composition de l'une quelconque des revendications précédentes, comprenant au moins 2 000 UI au total de cholécalciférol et d'ergocalciférol.

7. Composition de l'une quelconque des revendications précédentes, comprenant moins de 50 000 UI au total de cholécalciférol et d'ergocalciférol.

8. Composition de l'une quelconque des revendications précédentes, comprenant 10 000 UI ou moins au total de cholécalciférol et d'ergocalciférol.

9. Composition de l'une quelconque des revendications précédentes, comprenant 5 000 UI ou moins au total de cholécalciférol et d'ergocalciférol.

10. Composition de l'une quelconque des revendications 1 à 3, comprenant une quantité totale de cholécalciférol et d'ergocalciférol dans une plage de 500 UI à 50 000 UI.

11. Composition de l'une quelconque des revendications 1 à 3, comprenant 1 500 UI de cholécalciférol et 1 500 UI d'ergocalciférol.

12. Composition de l'une quelconque des revendications 1 à 10, dans laquelle l'enrobage entérique est résistant à la désintégration dans les sucs gastriques et se dissout partiellement à un pH dans une plage de 7,0 à 8,0.

13. Composition de l'une quelconque des revendications 1 à 11, dans laquelle l'enrobage entérique comprend de l'acétate-phtalate de cellulose.

14. Composition de l'une quelconque des revendications 1 à 11, comprenant en outre un agent calcimimétique.

15. Composition de l'une quelconque des revendications 1 à 11, dans laquelle la matrice est une matrice de cire.

16. Composition de la revendication 15, dans laquelle la matrice de cire comprend un mélange de cires, facultativement avec l'ajout d'huiles.

17. Procédé de fabrication d'une formulation à libération contrôlée de cholécalciférol et/ou d'ergocalciférol, comprenant la dissolution d'une quantité souhaitée de cholécalciférol et/ou d'ergocalciférol dans un volume minimal d'éthanol absolu de grade USP, et le mélange de la solution avec un ou plusieurs excipients de grade pharmaceutique pour former une matrice solide, semi-solide ou liquide qui est sensiblement résistante à la digestion dans l'estomac, et se désintégrant progressivement dans l'intestin inférieur, ladite matrice étant ensuite confinée dans un enrobage entérique.

18. Composition de l'une quelconque des revendications 1 à 11, pour utilisation dans le traitement d'une insuffisance ou déficience en 25-hydroxyvitamine D chez un patient, la composition étant formulée de manière à être administrée par voie orale à un patient ayant une insuffisance ou déficience en 25-hydroxyvitamine D, telle que **caractérisé par** des taux sériques de 25-hydroxyvitamine D inférieurs à 30 ng/ml.

19. Composition pour utilisation de la revendication 18, les taux sériques totaux de cholécalciférol et d'ergocalciférol étant augmentés en une quantité de 10 nmol/l ou moins sur une période d'au moins 2 jours.

20. Composition pour utilisation de la revendication 18 ou 19, les taux sériques totaux de 25-hydroxyvitamine D₃ et 25-hydroxyvitamine D₂ étant augmentés en une quantité de 3 ng/ml ou moins combinée sur une période d'au moins 2 jours.

21. Composition pour utilisation de l'une quelconque des revendications 18 à 20, les taux sériques de 25-hydroxyvitamine D étant restaurés à au moins 30 ng/ml.

22. Composition pour utilisation de l'une quelconque des revendications 18 à 21, les taux sériques de 25-hydroxyvitamine D étant maintenus à au moins 30 ng/ml pendant une période d'au moins trois mois.

23. Composition pour utilisation de la revendication 22, les taux sériques de 25-hydroxyvitamine D étant maintenus à au moins 30 ng/ml pendant une durée d'au moins six mois.

24. Composition pour utilisation de l'une quelconque des revendications 18 à 23, comprenant en outre la co-administration d'un agent calcimimétique.

25. Kit comprenant :
(a) une composition à libération contrôlée stable comprenant du cholécalciférol dispersé dans une matrice solide, semi-solide ou liquide, ladite matrice confinée avec un enrobage entérique, où
la composition retarde la libération substantielle du cholécalciférol jusqu'à ce qu'il atteigne l'iléon d'un patient ; et
(b) une composition à libération contrôlée stable comprenant de l'ergocalciférol dispersé dans une matrice solide, semi-solide ou liquide, ladite matrice étant confinée avec un enrobage entérique, la composition retardant la libération substantielle de l'ergocalciférol jusqu'à ce qu'il atteigne l'iléon d'un patient.
